# EUROPEAN PATENT SPECIFICATION

(11) **EP 4 516 265 B1**
(45) Date of publication and mention of the grant of the patent: **08.07.2026**
(21) Application number: 23795668.5
(22) Date of filing: 28.04.2023
(51) Int. Cl.: A61F 2/24

(54) **TRANSCATHETER AORTIC VALVE REPLACEMENT APPARATUS HAVING BILATERAL LOCKING FUNCTION**
TRANSKATHETER-AORTENKLAPPENERSATZVORRICHTUNG MIT BILATERALER VERRIEGELUNGSFUNKTION
APPAREIL DE REMPLACEMENT DE VALVULE AORTIQUE TRANSCATHÉTER AYANT UNE FONCTION DE VERROUILLAGE BILATÉRAL

(30) Priority: 29.04.2022 CN 202210473085
(43) Date of publication of application: 05.03.2025
(73) Proprietor: Wuhan Weike Medical Technology Co. Ltd, Wuhan, Hubei 430000 (CN)
(72) Inventor: CHEN, Song, Lake New Technology Development Zone Wuhan, Hubei 430000 (CN); WANG, Xueli, Lake New Technology Development Zone Wuhan, Hubei 430000 (CN); ZHANG, Changdong, Lake New Technology Development Zone Wuhan, Hubei 430000 (CN); SUN, Ming, Lake New Technology Development Zone Wuhan, Hubei 430000 (CN); WU, Chunlin, Lake New Technology Development Zone Wuhan, Hubei 430000 (CN)
(74) Representative: Murgitroyd & Company
(86) International application number: PCT/CN2023/091878
(87) International publication number: WO 2023/208236

(56) References cited:
- WO-A1-2022/012569
- CN-A- 111 265 334
- CN-A- 111 329 621
- CN-A- 113 288 519
- US-A1- 2011 295 363
- US-A1- 2020 113 683

## Description

### TECHNICAL FIELD

The present invention relates to a technical field of structural design of an apparatus for interventional treatment with a prosthetic heart valve, in particular, to a transcatheter aortic valve replacement apparatus having bilateral locking function.

### BACKGROUND

With the aging of the population, the incidence rate of valvular heart disease is obviously increased. At present, conventional surgical treatment is still the treatment of choice for most patients with severe valvular lesions, but there is a high risk of high trauma, postoperative mortality rate and complications. In recent years, transcatheter valve implantation/repair has gradually matured and been widely used, especially transcatheter aortic valve implantation (TAVR/TAVI), which has sufficient evidence-based basis and greatly reduces trauma, and has been recommended by European and American guidelines for the management of valvular heart disease. This is a milestone in interventional treatment of valvular heart diseases.

Transcatheter aortic valve implantation (TAVI) is anew technique for interventional placement of prosthetic aortic valves. It was first reported by Dr. Criber in France in 2002, offering hope for the treatment of patients with severe aortic stenosis (AS) who lost surgical opportunity (e.g., patients over 80 years old) and opening a new page in the history of cardiovascular intervention. Over the next decade, with the improvement of instruments and the accumulation of experience, the TAVI technique are being refined. More than 500 cardiac centers in nearly forty countries have practiced this technique, with a total of over 150000 cases. In particular, the efficacy, feasibility, and safety of TAVI have been demonstrated after a series of registration studies and randomized controlled trials. TAVI technique has become the treatment of choice for patients with severe AS who cannot undergo surgical valve replacement. Clinically, the prosthetic bioprosthetic valves used for TAVI primarily includes an Edwards Sapien (available from Edwards Lifesciences, USA) which is to be implanted by balloon expansion and a Core Valve (available from Medtronic, USA) which is to be implanted by self-expansion. TAVI technique has made impressive progress intemationally. TAVI technique has been preliminarily applied in China and has a promising prospect. At present, the domestic heart valve instrument market is a market highly monopolized by foreign brands. Foreign-funded enterprises such as Edwards Lifesciences, Medtronic, LivaNova (acquired by Solin), St. Jude Medical (acquired by Abbott) and On-X account for about 85% of the market share. Among them, Edwards Lifesciences and Medtronic have complete product lines from mechanical valves and bioprosthetic valves to transcatheter interventional valves. At the same time, a number of domestic enterprises of medical instrument have emerged in China. At present, three domestic transcatheter aortic valves have been approved by China's CFDA, and are a Venus-A available from Venus Medtech, a J-valve available from Suzhou Jiecheng's and a VitaFlow available from MicroPort CardioFlow, respectively. However, none of the domestic enterprises has an absolute leading advantage.

According to the statistical analysis of the echocardiography database of hospital patients, in patients aged 65 to 74 years (49995 cases) and patients over 75 years (34671 cases), the detection rates of moderate or severe aortic regurgitation (AR) were 2.12% and 2.85% respectively, the detection rates of moderate or severe aortic stenosis (AS) were 0.75% and 0.89% respectively, and the detection rates of severe aortic regurgitation (SAR) and severe aortic stenosis (SAS) were 0.52 % VS 0.95% and 0.54% VS 0.57% respectively, in both aged groups. It can be seen that Chinese elderly people tend to have aortic regurgitation in degenerative aortic valvular diseases. There are some differences between the patients with aortic valve disease in China and those in western countries: (1) The proportion of patients with bicuspid aortic valve in China is high. The proportion of patients with bicuspid aortic valve is in a range of 40 %~50%, which is much higher than the 1.6 %~9.3% in western countries. Multiple large-scale TAVR clinical studies in Western countries have listed the bicuspid aortic valve as an exclusion criterion. (2) The calcification degree of aortic valve is higher in China. (3) In China, there are more patients with aortic valve regurgitation than with aortic valve stenosis. (4) The inner diameter of femoral artery is smaller, in China, the average inner diameter of femoral artery in the candidate cases of TAVR was 6.5 mm.

The Venus-A available from Venus Medtech and the VitaFlow available from MicroPort CardioFlow adopt percutaneous route and self-expansion stent design, and are mainly used for treating aortic valve stenosis. Although the J-valve available from Suzhou Jiecheng also adopts a design of a self-expansion stent and has three anchoring devices that secure the stent to the bottom of the native valve leaflet to address aortic regurgitation, the path adopted is transapical and the transapical path is more traumatic than the percutaneous path. Meanwhile, products of the Venus-A available from Venus Medtech and the J-valve available from Suzhou Jiecheng adopt porcine pericardium, and the VitaFlow available from MicroPort CardioFlow adopts bovine pericardium which has better durability than porcine pericardium. To sum up, the three aortic valve products currently on the market in China all have certain limitations in the treatment of disease or their own performance deficiencies.

In the Chinese patent application No. 2021107458307, there is provided a transcatheter aortic valve device for automatically capturing valve leaflets. The device has a stent, a valve skirt, and a positioning member. The positioning member is an extended part of the stent, and the connection between the positioning member and the stent also forms a release structure of the stent. In actual use, the connection between the stent and the positioning member is used to release the stent. In the clinical operation process, it is found that, when the above technique is used, the connection between the stent and the positioning member may affect the positioning of the positioning member in the stent release process, resulting in inaccurate positioning of the device, adverse surgical consequences, and even endangering the life safety of the patient in serious cases. In addition, as blood and cardiac tissue exert a large force on the stent, relative displacement occurs between the stent and the positioning member, thereby causing the positioning member to be pulled and affecting its lifespan.

CN 111 329 621 discloses a transcatheter aortic artificial valve that comprises grid-shaped support which can be compressed and expanded in radial direction, and artificial valve leaf mounted on inner side of support comprising inflow end and outflow end

### SUMMARY

Based on the above description, the present invention provides a transcatheter aortic valve replacement apparatus having bilateral locking function, so as to solve the technical problems in the prior art that the positioning of the positioning member may be affected in the stent release process due to unreasonable structural design of the aortic valve product and thus the positioning of the apparatus is inaccurate.

The technical solution for solving the above technical problems is as follows.

A transcatheter aortic valve replacement apparatus having bilateral locking function, includes a self-expansion stent, a valve skirt, a valve leaflet, at least two elastic positioning members, an outflow end release member, and an inflow end release member.

A through-hole for liquid circulation is defined within the self-expansion stent. The self-expansion stent has an inflow end and an outflow end opposite to the inflow end corresponding to the through-hole. A sidewall of the self-expansion stent has a grid-shaped structure. The valve skirt is connected to a sidewall of the through-hole. The valve leaflet is connected to an inner side of the valve skirt. A sidewall of the self-expansion stent adjacent to the outflow end is formed by a plurality of outflow end grids arranged in an annular shape.

Each elastic positioning member has a U-shaped portion and connecting arms located at two ends of the U-shaped portion. Each connecting arm is connected to the outflow end. The U-shaped portion is located on an outer side of the self-expansion stent. A middle part of the U-shaped portion extends toward the inflow end and is elastically foldable.

The outflow end release member is connected to the outflow end of the self-expansion stent and located between two of the elastic positioning members. The inflow end release member is connected to the inflow end of the self-expansion stent.

Compared with the prior art, the technical solution of the present application has the following beneficial technical effects.

In the transcatheter aortic valve replacement apparatus provided in this invention, the elastic positioning member and the outflow end release member are provided on the stent, and the positioning of the stent and the release of the outflow end are separate in function, and thus are independent from each other and do not affect each other, which effectively avoids the effective mounting of the positioning member and even the entire self-expansion stent from being affected when the stent is released by the outflow end release member. In addition, the outflow end release member is mounted between the elastic positioning members, which effectively ensures the compactness and stability of the whole apparatus. The outflow end release member and the inflow end release member can be fixed on the delivery system at both sides, and can be more conveniently mounted inside the delivery system.

On the basis of the above technical solution, this invention can also be improved as follows.

Further, the connecting arm is connected to the outflow end grid or the outflow end release member at a first connection point. The outflow end release member is connected to the outflow end grid at a second connection point. At least one of the first connection point and the second connection point is staggered from a vertex of the outflow end grid.

After the above technical solution is adopted, one of the first connection point and the second connection point is separated from the vertex of the outflow end grid, and the force forcing relative displacement on the connection between the elastic positioning member and the self-expansion stent can be effectively dispersed, which can effectively protect the elastic positioning member from fracturing.

Further, an included angle between the connecting arm and a plane tangent to the corresponding outflow end grid is in a range of 0°~45°.

Further, the connecting arm is located on the plane tangent to the corresponding outflow end grid.

After the above technical solution is adopted, the radial pressure received by the elastic positioning member can be effectively transferred to the self-expansion stent, and then the self-expansion stent would bear such force, so as to counteract the radial force borne by the positioning member.

Further, three elastic positioning members are provided. The three elastic positioning members are evenly distributed in a circumferential direction of the self-expansion stent.

Further, repair parts are formed near two ends of the U-shaped portion. The transcatheter aortic valve replacement apparatus further includes a repair member. Each repair part is connectable to the self-expansion stent through the repair member.

Further, the repair part and the first connection point are located at two ends of the connecting arm. The repair member has a soft sheet-like structure. The repair member is capable of wrapping or winding around the connecting arm in a length direction of the connecting arm. Two ends of the repair member corresponding to the connecting arm are fixed to the corresponding first connection point and the corresponding repair part, respectively.

After the above technical solution is adopted, the repair member is wound around the connecting arm and fixed, which further enhance the connection between the connecting arm and the self-expansion stent and the U-shaped portion. When any end of the elastic positioning member disengages from the self-expansion stent, the elastic positioning member can be restricted by the repair member to avoid further damage to the patient's body.

Further, the repair member has an elongated strip structure and one repair member is provided. An end of the repair member is capable of extending through all of the repair parts in sequence and being connected to the self-expansion stent.

Further, the repair member has an elongated strip structure and the number of the repair members is equal to the number of the elastic positioning members. An end of each of the repair members extends through the repair parts on the same U-shaped portion in sequence and is connected to the self-expansion stent.

After the above technical solution is adopted, the connection relationship between the elastic positioning member and the self-expansion stent can be repaired in time by the repair member when the elastic positioning member falls off or is connected unstably, which can prevent the patient from being further damaged.

Further, the transcatheter aortic valve replacement apparatus further includes a protective member connected to an outer side of a middle part of the U-shaped portion by suturing, wrapping, or winding.

Further, the repair member and the protective member are polyester cloth or a bovine pericardium.

After the above technical solution is adopted, the U-shaped portion is covered by the protective member during use, which can effectively prevent the U-shaped portion from scratching the cardiac tissue or blood vessel wall.

Further, the outflow end release member includes a release portion and support arms located on two sides of the release portion. The support arm is connected to the self-expansion stent at the second connection point. Two second connection points of the same outflow end release member are located at vertexes of different outflow end grids. The release portion extends away from the outflow end in the first direction.

After the above technical solution is adopted, the outflow end release member and the elastic positioning member respectively extend in two directions, so that the interplay between the two is further reduced.

Further, a release grid is enclosed by the support arms of each outflow end release member and adjacent outer sides of two outflow end grids. An end of the valve leaflet adjacent to the inflow end is sutured to the valve skirt in a circumferential direction, and another end of the valve leaflet is connected to the valve skirt at a position inside the release grid.

After the above technical solution is adopted, the release grid is enclosed by the support arms and the adjacent outer sides of the two outflow end grids, and the end of the valve leaflet adjacent to the outflow end is connected to the valve skirt at the position inside the release grid, so that the size of the release grid is much greater than the area available at the connection in the prior art, such that t the valve leaflet is bonded to the valve skirt more conveniently and firmly, and the service life of the apparatus is prolonged.

Further, a hole size of the through-hole gradually becomes smaller and then remains unchanged or gradually becomes larger in a first direction, and the release grid is bent toward an inner side of the through-hole in the first direction.

Further, the elastic positioning member and the self-expansion stent are formed by cutting a nickel-titanium alloy pipe in a single pass, followed by thermoforming.

After the above technical solution is adopted, the stability of the structural connection between the self-expansion stent and the elastic positioning member is ensured.

Further, a sidewall of the self-expansion stent adjacent to the inflow end is formed by a plurality of inflow end grids arranged in an annular shape. The inflow end release member includes an elliptical structure connected to a vertex of one of the inflow end grids.

After the above technical solution is adopted, since the elliptical structure has no sharp end, the outer side of the elliptical structure is a smooth curved surface, which reduces the space for the movement of the inflow end release member and prevents the vascular wall from being scratched.

Further, a penetration hole is formed at a middle part of the elliptical structure in a radial direction of the through-hole.

After the above technical solution is adopted, the whole elliptical structure is ring-shaped. compared with a solid elliptical structure, this elliptical structure is softer as a whole, and it is not easy to puncture human organs and tissues.

Further, an extension line of a short axis of the elliptical structure is parallel to an axis of the through-hole, and an extension line of a long axis of the elliptical structure is located on a plane tangent to the corresponding inflow end grid.

After the above technical solution is adopted, the inflow end grid is designed to have a vertical contraction form at the apex, which can effectively prevent the aortic vessel wall from being scratched during the mounting of the stent.

### BRIEF DESCRIPTION OF THE DRAWINGS

FIG. 1 is a perspective schematic view illustrating a structure of a transcatheter aortic valve replacement apparatus having bilateral locking function according to an embodiment of the present invention.
FIG. 2 is a schematic view illustrating the structure in FIG. 1 with the valve skirt and the valve leaflet omitted.
FIG. 3 is a schematic view illustrating a connection structure, with a first connection point, a second connection point coinciding with a vertex of an outflow end grid.
FIG. 4 is a schematic view illustrating a structure of an embodiment adopting a first staggered setting of the present invention.
FIG. 5 is a schematic view illustrating a structure of an embodiment adopting a second staggered setting of the present invention.
FIG. 6 is a top schematic view illustrating a structure in which a support arm and a self-expansion stent are disposed at a certain angle.
FIG. 7 is a top schematic view illustrating a structure in which a support arm and a self-expansion stent are tangent to each other.
FIG. 8 is a schematic view illustrating a structure of another embodiment having a repair part compared with the embodiment shown in FIG. 5.
FIG. 9 is a schematic view illustrating a structure of a repair part in another embodiment of the present invention.
FIG. 10 is a schematic view illustrating a compressed structure according to an embodiment of the present invention.

### DETAILED DESCRIPTION

In order to facilitate the understanding of the present application, a more comprehensive description of the present application will be provided below with reference to the relevant drawings. Embodiments of the present application are illustrated in the drawings. However, the present application may be implemented in many different forms and is not limited to the embodiments described herein. Rather, these embodiments are provided for the purpose of making the disclosure of the present application more thorough and comprehensive.

Unless otherwise defined, all technical and scientific terms used herein have the same meanings as those commonly understood by those skilled in the art to which the present application belongs. The terms used in the description of the present application herein are only for the purpose of describing specific embodiments and are not intended to limit the present application.

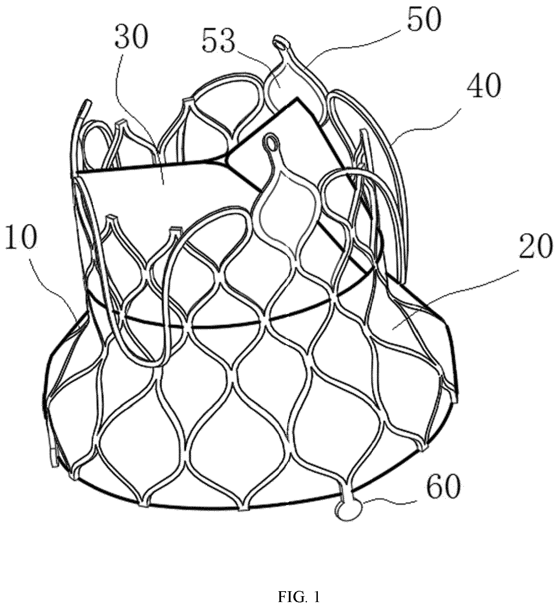

As shown in FIG. 1, the present application provides a transcatheter aortic valve replacement apparatus having bilateral locking function, including a self-expansion stent 10, a valve skirt 20, a valve leaflet 30, at least two elastic positioning members 40, and at least two outflow end release members 50.

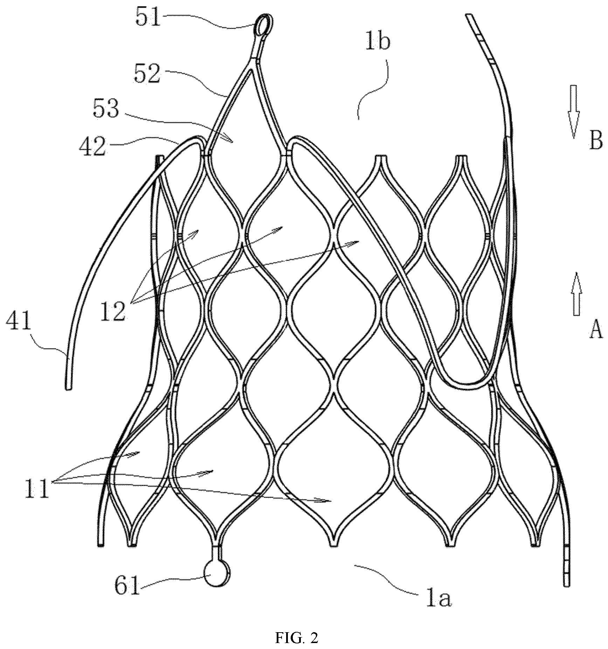

In the present embodiment, the self-expansion stent 10 defines a through-hole therewithin for liquid (e.g., blood) circulation. The self-expansion stent 10 has an inflow end 1a and an outflow end 1b opposite to the inflow end 1a, corresponding to the through-hole. The sidewall of the self-expansion stent 10 has a grid-shaped structure. The valve skirt 20 is connected to the sidewall of the through-hole. The valve leaflet 30 is connected to the inner side of the valve skirt 20. As shown in FIG. 2, the valve leaflet 30 is configured to allow blood to flow through the through-hole in a first direction A from the inflow end 1a to the outflow end 1b, and prevents blood from flowing through the through-hole in a second direction B opposite to the first direction A. The sidewall of the self-expansion stent 10 adjacent to the inflow end 1a is formed by a plurality of inflow end grids 11 arranged in an annular shape. The sidewall of the self-expansion stent 10 adjacent to the outflow end 1b is formed by a plurality of outflow end grids 12 arranged in an annular shape.

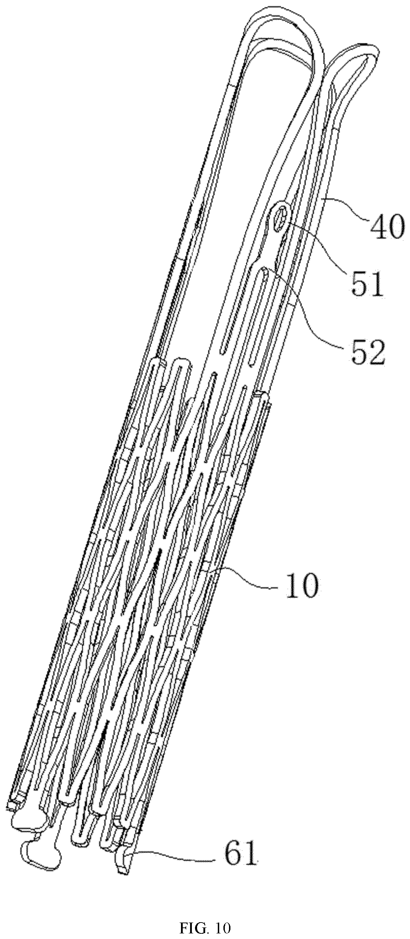

As shown in FIG. 2, the elastic positioning member 40 has a U-shaped portion 41 and connecting arms 42 located at two ends of the U-shaped portion 41. The U-shaped portion 41 is located on the outer side of the self-expansion stent 10. The middle part of the U-shaped portion 41 extends toward the inflow end 1a and can be elastically folded. It is well known that when the self-expansion stent 10 is fed into the human body, the self-expansion stent 10 needs to be compressed. As shown in FIG. 10, the U-shaped portion 41 can be elastically folded to extend toward the outflow end.

The outflow end release member 50 is connected to the outflow end 1b of the self-expansion stent 10 and located between the two elastic positioning members 40. The connecting arm 42 is connected to the outflow end grid 12 or the outflow end release member 50 at a first connection point L1. The outflow end release member 50 is connected to the outflow end grid 12 at a second connection point L2.

In this embodiment, the elastic positioning of the self-expansion stent 10 and the release of the outflow end 12 are separate in function, and thus are independent from each other and do not affect each other. Therefore, it can effectively avoid that the heart valve cannot be accurately positioned due to the fact that the self-expansion stent 10 may affect the positioning and mounting of the elastic positioning member 40 during the release of the outflow end. In addition, the outflow end release member 50 is mounted between the elastic positioning members 40, which effectively ensures the compactness and stability of the whole apparatus.

In the present application, the outflow end release member 50 includes a release portion 51 and support arms 52 located on two sides of the release portion. The second connection point L2 is formed by connecting the support arm 52 to the self-expansion stent 10. The two second connection points L2 of the same outflow end release member 50 are located at the vertexes of different outflow end grids 12, respectively. The release portion 51 extends away from the outflow end 1b in the first direction.

The outflow end release member 50 may be considered as a part extending from or connected to the self-expansion stent 10. The elastic positioning member 40 may be connected to the outflow end grid 12 of the self-expansion stent 10, or may be connected to the two support arms 52.

The release portion 51 is configured to be connected to a delivery system that delivers the valve apparatus into the human body. When the release portion 51 is disconnected from the delivery system, driven by the self-expansion stent 10, the outflow end release member 50 expands outwardly, until the outflow end 1b is released. After the mounting is completed, the outflow end release member 50 and the elastic positioning member 40 respectively extend in two directions, so that the interplay between the two is further reduced.

More preferably, a release grid 53 is enclosed by the support arms 52 of each outflow end release member 50 and the adjacent outer sides of two outflow end grids 12. An end of the valve leaflet 30 adjacent to the inflow end 1a is sutured to the valve skirt 20 in a circumferential direction, and another end of the valve leaflet 30 is connected to the valve skirt 20 at a position inside the release grid 53. The release grid 53 is enclosed by the support arms 52 and the adjacent outer sides of the two outflow end grids 12, and the end of the valve leaflet 30 adjacent to the outflow end 12 is connected to the valve skirt 20 at the position inside the release grid 53, so that the size of the release grid 53 is much greater than the area available at the connection in the prior art, such that the valve leaflet 30 is bonded to the valve skirt 20 more conveniently and firmly, and the service life of the heart valve apparatus is prolonged.

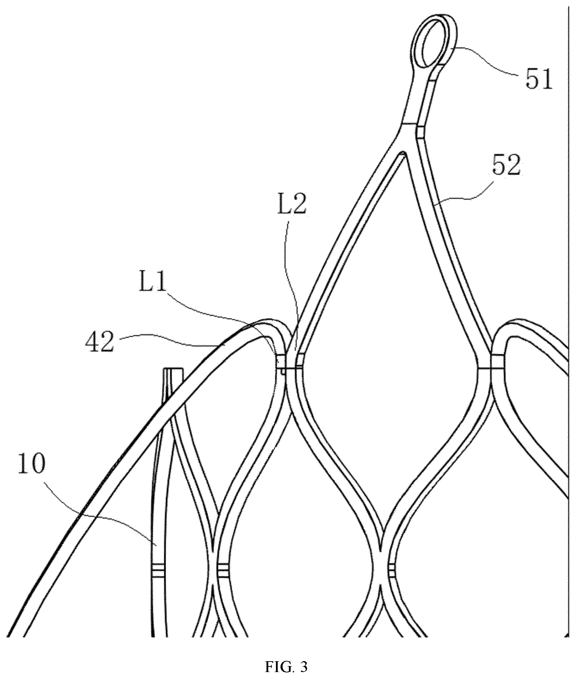

For the connection mode between the elastic positioning member 40 and the outflow end release member 50, the most obvious and most easily conceived mode is shown in FIG. 2 and FIG. 3, that is, the first connection point L1 and the second connection point L2 are both at the vertex of the outflow end grid 12.

After the force analysis and research on the transcatheter aortic valve replacement apparatus during use, it is found that the self-expansion stent 10 and the outer sidewall of the elastic positioning member 40 are in contact with the cardiac tissue during use. Firstly, the elastic positioning member 40 needs to bear the pressure from the cardiac tissue in the radial direction of the self-expansion stent 10. At the same time, due to the frictional force generated when the blood flows through the interior of the through-hole, and the swirling force due to uneven pressure of the blood, the self-expansion stent 10 and the elastic positioning member 40 tend to rotate relative to each other, thereby forming a rotational pulling force. In addition, because the end of the valve leaflet 30 adjacent to the outflow end 12 is connected to the valve skirt 20 at the position inside the release grid 53, when the blood flows through the through-hole, the valve leaflet 30 is periodically opened and closed, so that the regions near the outflow end grid 12 are periodically swung, thereby forming a periodic swinging pulling force on the elastic positioning member 40. When the first connection point L1, the second connection point L2 and the vertex of the outflow end grid 12 are clustered together, the above-mentioned several forces will act on the elastic positioning member 40, forcing the elastic positioning member 40 and the self-expansion stent 10 to move relative to each other and generating a large pulling force therebetween, which increases the risk of fracture of the elastic positioning member 40. If both the first connection point L1 and the second connection point L2 coincide with the vertex of the outflow end grid 12, the fracture risk will be greatly increased. Therefore, in the present application, the more dispersed the first connection point L1, the second connection point L2 and the vertex of the outflow end grid, the less pulling force the elastic positioning member 40 would be subjected to, which effectively reduces the risk of fracture of the two ends of the elastic positioning member 40. Preferably, at least one of the first connection point L1 and the second connection point L2 is staggered from the vertex of the corresponding outflow end grid 12.

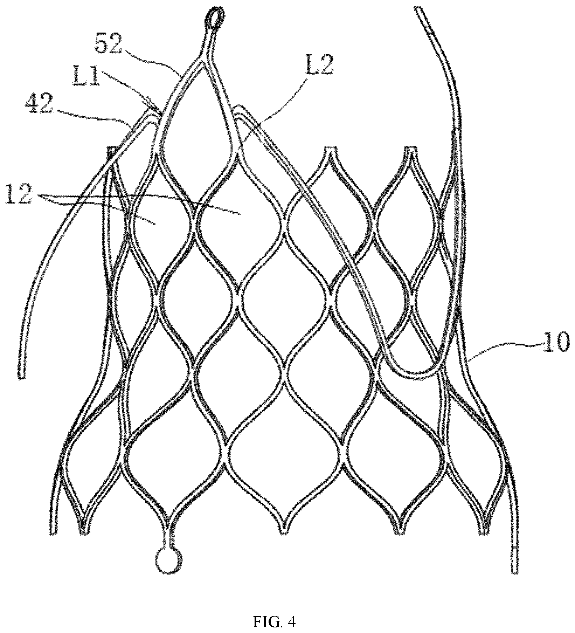

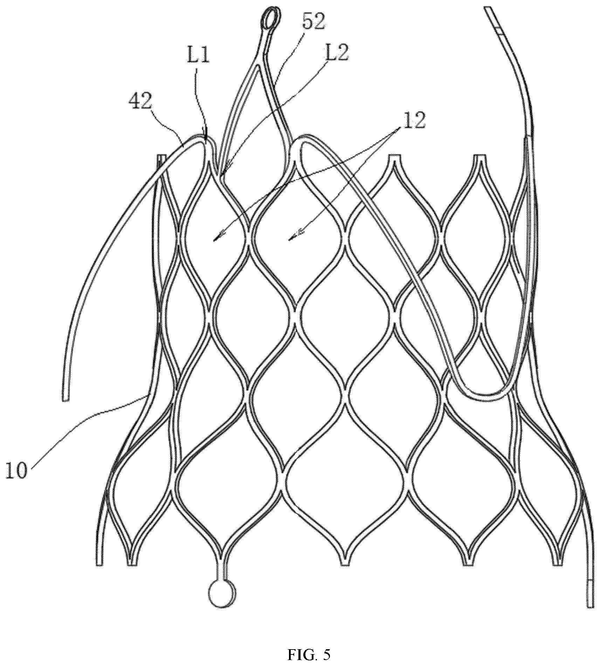

Obviously, the above staggered setting includes three cases. The first case is shown in FIG. 4, that is, the second connection point L2 coincides with the vertex of the outflow end grid 12, and the first connection point L1 is staggered from such coinciding position. In this case, the connecting arm 42 can be directly connected to the support arm 52. The second case is shown in FIG. 5, that is, the first connection point L1 coincides with the vertex of the outflow end grid 12, and the second connection point L2 is staggered from such coinciding position. The third case is that both the first connection point L1 and the second connection point L2 are staggered from the vertex of the outflow end grid 12.

For each one of the connection cases as described above, one of the first connection point and the second connection point is effectively separate, so as to disperse the force and prevent the elastic positioning member 40 from fracturing.

In a preferred embodiment of the present application, three elastic positioning member 40 and three outflow end release member 50 are provided. The three elastic positioning members and the three outflow end release members 50 are evenly distributed in the circumferential direction of the self-expansion stent 10.

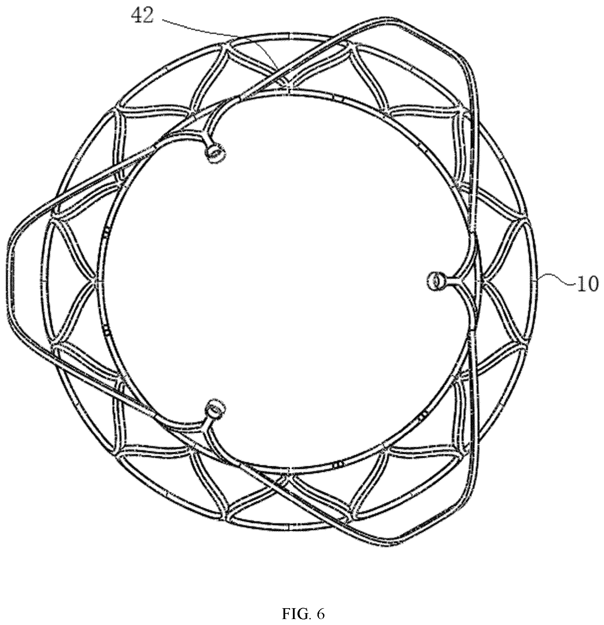

The relative included angle between the connecting arm 42 and the outflow end grid 12 can meet the use requirements within a certain range of the included angle. Specifically, the included angle between the connecting arm 42 and the plane tangent to the corresponding outflow end grid 12 is in a range of 0°-45°. In the top view shown in FIG. 6, the included angle between the connecting arm 42 and the plane tangent to the self-expansion stent 10 is 45°.

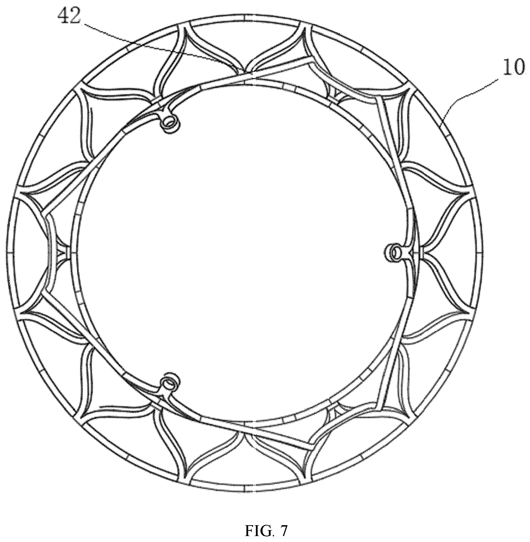

According to the fracture analysis of the elastic positioning member 40, it is found that the main fracture effect comes from the radial pressure. Therefore, it is preferred that the connecting arm 42 is tangent to the self-expansion stent 10. When the cardiac tissue exerts the radial pressure on the elastic positioning member 40, such structure will release the radial pressure and transfer the radial pressure to the self-expansion stent 10. As such, the self-expansion stent would bear such acting force, so as to achieve the effect that the elastic positioning member 40 can counteract the radial pressure. Therefore, in this embodiment, as shown in FIG. 7, the connecting arm 42 is located on the plane tangent to the corresponding self-expansion stent 10, that is, the included angle between the connecting arm 42 and the plane tangent to the corresponding outflow end grid 12 is 0.

Based on the above description, the present application can effectively reduce the risk of the elastic positioning member being broken by dispersing the force on the one hand, and by counteracting the force on the other hand, which greatly prolongs the service life of the heart valve.

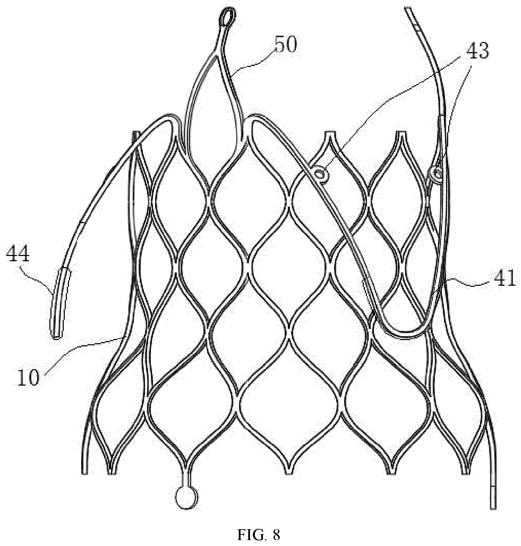

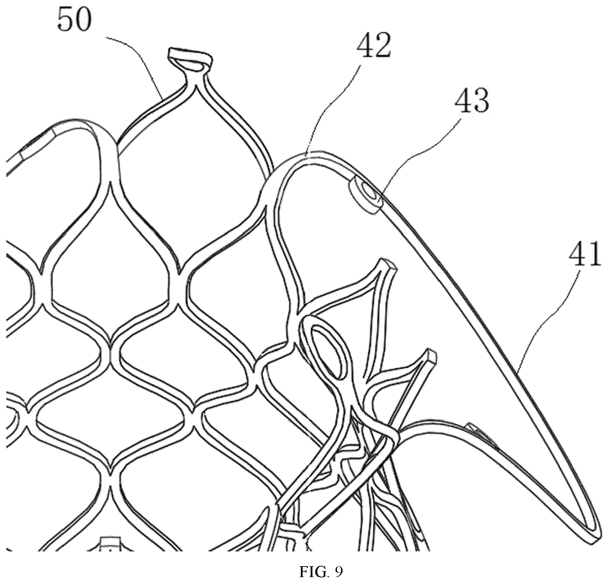

However, in the practical use of the heart valve, with the lengthening of the use time and the aging of the material, the elastic positioning member 40 and the self-expansion stent 50 may still fracture and fall off. If the elastic positioning member 40 is allowed to move freely in the body, the elastic positioning member 40 may puncture the heart, which may cause serious irreversible damage to the patient's body. Therefore, an effective repair means needs to be provided. As shown in FIG. 8 and FIG. 9, in another preferred embodiment of the present application, repair parts 43 are formed near both ends of the U-shaped portion 41. The transcatheter aortic valve replacement apparatus further includes a repair member (not shown in the figures). The repair part 43 may be connected to the self-expansion stent 10 through the repair member. In some embodiments, the repair part 43 is a thick part having holes and formed at the ends of the U-shaped portion 41. In other embodiments, the repair parts are lugs formed at the ends of the U-shaped portion 41, which may be in a shape of a closed loop or may be a hook bent inward.

The repair part 43 and the first connection point L1 are located at two ends of the connecting arm 42. In some embodiments, the repair member has a soft sheet-like structure. The repair member can wrap or wind around the connecting arm 42 in the length direction of the connecting arm 42. The two ends of the repair member corresponding to the connecting arm are fixed to the corresponding first connection point L1 and the corresponding repair part 43, respectively.

Preferably, in the above embodiment, the repair member is a polyester cloth or a bovine pericardium.

In some other embodiments, the repair member may have an elongated strip structure, specifically, may be a rope or a nickel-titanium wire, and one repair member is provided. An end of the repair member may extend through all repair parts 43 in sequence and be connected to the self-expansion stent 10. In some other embodiments, the number of repair members is equal to the number of elastic positioning members 40. An end of each repair member extends through the repair parts 43 on the same U-shaped portion 41 in sequence, and is connected to the self-expansion stent 10. The above connection may optionally be hooked, wound, or bound to the self-expansion stent 10, so as to ensure that the U-shaped portion 41 does not disengage from the self-expansion stent 10.

After the above technical solution is adopted, the connection relationship between the elastic positioning member 40 and the self-expansion stent 10 can be repaired in time by the repair member when the elastic positioning member 40 falls off or is connected unstably, so as to prevent the patient from being injured.

In a preferred embodiment of the present application, the transcatheter aortic valve replacement apparatus further includes a protective member 44. The protective member 44 is connected to the outer side of the middle part of the U-shaped portion 41 by suturing, wrapping, or winding. The protective member 44 is preferably a polyester cloth or a bovine pericardium.

In the embodiment of the present application, the hole size of the through-hole gradually becomes smaller and then remains unchanged or gradually becomes larger in the first direction A. Such trumpet-shaped opening can make the self-expansion stent 10 be well fixed at the aortic valve of the human body. The release grid 53 is bent toward the inner side of the through-hole in the first direction A. As an optional connection mode, the release portion 51 has an annular structure. The delivery system and the release portion 51 are connected to each other by a strip-shaped connecting piece having a coil. The coil can pass into the other side of the release portion 51 in the second direction. The strip-shaped piece, which can be axially movable, extends through the coil to limit the position of the release portion 51. When the strip-shaped piece is withdrawn from the coil, the release portion 51 can be disengaged from the outside of the coil under the effect of elasticity to complete the release of the outflow end. Since the release grid 53 is bent inwardly in the first direction A, the whole strip-shaped piece may be located inside the tubular body formed after the self-expansion stent is compressed, and the placement of the strip-shaped piece may prevent the cardiac tissue from be punctured.

In order to ensure the stability of the structural connection between the self-expansion stent 10 and the elastic positioning member 40, the elastic positioning member 40 and the self-expansion stent 10 are formed by cutting a nickel-titanium alloy pipe in a single pass and then thermoforming it.

To achieve accurate release and mounting of the inflow end, as shown in FIG. 1 and FIG. 2, the transcatheter aortic valve replacement apparatus further includes a plurality of inflow end release members 60. The inflow end release member 60 includes an elliptical structure 61 connected to the vertex of the inflow end grid 11. Since the elliptical structure 61 has no sharp end, the outer side of the elliptical structure 61 is a smooth curved surface, which reduces the space for the movement of the inflow end release member 60 and prevents the vascular wall from being scratched. In order to further strengthen the soft characteristics of the elliptical structure, a penetration hole is formed at its middle part in the radial direction of the through-hole. In this way, the elliptical structure 61 is ring-shaped as a whole. The solid elliptical structure is very hard and protrudes outwardly relative to the inflow end, and easily punctures the cardiac tissue. The elliptical structure 61 is provided with a ring-shaped structure and cooperate with the nickel-titanium alloy material, to be more soft, so that the human organs and tissues are not easy to be punctured. In addition, both the inflow end release member 60 and the outflow end release member 50 that are ring-shaped can be fixed on the delivery system at both sides, without affecting the mounting and releasing function of the stent.

In a preferred embodiment, the extension line of the short axis of the elliptical structure 61 is parallel to the axis of the through-hole, and the extension line of the long axis of the elliptical structure 61 is located on the plane tangent to the corresponding inflow end grid 11. In this way, the inflow end grid 11 is designed to have a vertical contraction form at the vertex, which can effectively prevent the aortic vessel wall from being scratched during the mounting of the transcatheter aortic valve replacement apparatus.

The above description are only preferred embodiments of the present invention and is not intended to limit the present invention. The invention is disclosed by the appended claims.

## Claims

1. A transcatheter aortic valve replacement apparatus having bilateral locking function, comprising a self-expansion stent (10), a valve skirt (20), a valve leaflet (30), at least two elastic positioning members (40), an outflow end release member (50), and an inflow end release member (60);
wherein a through-hole for liquid circulation is defined within the self-expansion stent (10); the self-expansion stent (10) has an inflow end (1a) and an outflow end (1b) opposite to the inflow end (1a), corresponding to the through-hole; and a sidewall of the self-expansion stent (10) has a grid-shaped structure; wherein the valve skirt (20) is connected to a sidewall of the through-hole; wherein the valve leaflet (30) is connected to an inner side of the valve skirt (20); a sidewall of the self-expansion stent (10) adjacent to the outflow end (1b) is formed by a plurality of outflow end grids (12) arranged in an annular shape;
each elastic positioning member (40) has a U-shaped portion (41) and connecting arms (42) located at two ends of the U-shaped portion (41); wherein each connecting arm (42) is connected to the outflow end (1b); the U-shaped portion (41) is located on an outer side of the self-expansion stent (10); and a middle part of the U-shaped portion (41) extends toward the inflow end (1a) and is elastically foldable; and
wherein the outflow end release member (50) is connected to the outflow end (1b) of the self-expansion stent (10) and located between two of the elastic positioning members (40); and the inflow end release member (60) is connected to the inflow end (1a) of the self-expansion stent (10).

2. The transcatheter aortic valve replacement apparatus having bilateral locking function according to claim 1, wherein the connecting arm (42) is connected to the outflow end grid (12) or the outflow end release member (50) at a first connection point (L1); the outflow end release member (50) is connected to the outflow end grid (12) at a second connection point (L2); and at least one of the first connection point (L1) and the second connection point (L2) is staggered from a vertex of the outflow end grid (12).

3. The transcatheter aortic valve replacement apparatus having bilateral locking function according to claim 1, wherein an included angle between the connecting arm (42) and a plane tangent to the corresponding outflow end grid (12) is in a range of 0°~45°;
and optionally wherein the connecting arm (42) is located on the plane tangent to the corresponding outflow end grid (12).

4. The transcatheter aortic valve replacement apparatus having bilateral locking function according to claim 1, wherein three elastic positioning members (40) are provided, and the three elastic positioning members (40) are evenly distributed in a circumferential direction of the self-expansion stent (10).

5. The transcatheter aortic valve replacement apparatus having bilateral locking function according to any one of claims 1 to 4, wherein repair parts (43) are formed near two ends of the U-shaped portion (41);
the transcatheter aortic valve replacement apparatus further comprises a repair member, and wherein each repair part (43) is connectable to the self-expansion stent (10) through the repair member.

6. The transcatheter aortic valve replacement apparatus having bilateral locking function according to claim 5, wherein the repair part (43) and the first connection point (L1) are located at two ends of the connecting arm (42); the repair member has a soft sheet-like structure; the repair member is capable of wrapping or winding around the connecting arm (42) in a length direction of the connecting arm (42); and two ends of the repair member corresponding to the connecting arm (42) are fixed to the corresponding first connection point (L1) and the corresponding repair part (43), respectively.

7. The transcatheter aortic valve replacement apparatus having bilateral locking function according to claim 5, wherein the repair member has an elongated strip structure and one repair member is provided; an end of the repair member is capable of extending through all of the repair parts (43) in sequence and being connected to the self-expansion stent (10); or
wherein the repair member has an elongated strip structure; and the number of the repair members is equal to the number of the elastic positioning members (40); and an end of each of the repair members extends through the repair parts (43) on the same U-shaped portion (41) in sequence and is connected to the self-expansion stent (10).

8. The transcatheter aortic valve replacement apparatus having bilateral locking function according to claim 6, further comprising a protective member (44) connected to an outer side of a middle part of the U-shaped portion (41) by suturing, wrapping, or winding;
and optionally wherein the repair member and the protective member (44) are polyester cloth or a bovine pericardium.

9. The transcatheter aortic valve replacement apparatus having bilateral locking function according to claim 2, wherein the outflow end release member (50) comprises a release portion (51) and support arms (52) located on two sides of the release portion (51); the support arm (52) is connected to the self-expansion stent (10) at the second connection point (L2); two second connection points (L2) of the same outflow end release member (50) are located at vertexes of different outflow end grids (12); and the release portion (51) extends away from the inflow end (1a).

10. The transcatheter aortic valve replacement apparatus having bilateral locking function according to claim 9, wherein a release grid (53) is enclosed by the support arms (52) of each outflow end release member (50) and adjacent outer sides of two outflow end grids (12); an end of the valve leaflet (30) adjacent to the inflow end (1a) is sutured to the valve skirt (20) in a circumferential direction, and another end of the valve leaflet (30) is connected to the valve skirt (20) at a position inside the release grid (53).

11. The transcatheter aortic valve replacement apparatus having bilateral locking function according to claim 10, wherein a hole size of the through-hole gradually becomes smaller and then remains unchanged or gradually becomes larger in a first direction (A), and the release grid (53) is bent toward an inner side of the through-hole in the first direction (A).

12. The transcatheter aortic valve replacement apparatus having bilateral locking function according to claim 10, wherein the elastic positioning member (40) and the self-expansion stent (10) are formed by cutting a nickel-titanium alloy pipe in a single pass, followed by thermoforming.

13. The transcatheter aortic valve replacement apparatus having bilateral locking function according to claim 1, wherein a sidewall of the self-expansion stent (10) adjacent to the inflow end (1a) is formed by a plurality of inflow end grids (11) arranged in an annular shape; and the inflow end release member (60) comprises an elliptical structure (61) connected to a vertex of one of the inflow end grids (11).

14. The transcatheter aortic valve replacement apparatus having bilateral locking function according to claim 13, wherein a penetration hole is formed at a middle part of the elliptical structure (61) in a radial direction of the through-hole.

15. The transcatheter aortic valve replacement apparatus having bilateral locking function according to claim 13, wherein an extension line of a short axis of the elliptical structure (61) is parallel to an axis of the through-hole, and an extension line of a long axis of the elliptical structure (61) is located on a plane tangent to the corresponding inflow end grid (11).

## Patentansprüche

1. Eine Transkatheter-Aortenklappenersatzvorrichtung mit bilateraler Verriegelungsfunktion, die einen selbstexpandierenden Stent (10), eine Klappenschürze (20), ein Klappensegel (30), mindestens zwei elastische Positionierungselemente (40), ein Ausströmende-Freigabeelement (50) und ein Einströmende-Freigabeelement (60) beinhaltet;
wobei ein Durchgangsloch zur Flüssigkeitszirkulation innerhalb des selbstexpandierenden Stents (10) definiert ist; der selbstexpandierende Stent (10) ein Einströmende (1a) und ein Ausströmende (1b) gegenüber dem Einströmende (1a) aufweist, das dem Durchgangsloch entspricht; und eine Seitenwand des selbstexpandierenden Stents (10) eine gitterförmige Struktur aufweist; wobei die Klappenschürze (20) mit einer Seitenwand des Durchgangslochs verbunden ist; wobei das Klappensegel (30) mit einer Innenseite der Klappenschürze (20) verbunden ist; eine Seitenwand des selbstexpandierenden Stents (10) angrenzend an das Ausströmende (1b) durch eine Vielzahl von Ausströmende-Gittern (12) gebildet ist, die in einer Ringform angeordnet sind;
jedes elastische Positionierungselement (40) einen U-förmigen Abschnitt (41) und Verbindungsarme (42) aufweist, die sich an zwei Enden des U-förmigen Abschnitts (41) befinden; wobei jeder Verbindungsarm (42) mit dem Ausströmende (1b) verbunden ist; der U-förmige Abschnitt (41) sich auf einer Außenseite des selbstexpandierenden Stents (10) befindet; und ein Mittelteil des U-förmigen Abschnitts (41) sich zum Einströmende (1a) erstreckt und elastisch faltbar ist; und
wobei das Ausströmende-Freigabeelement (50) mit dem Ausströmende (1b) des selbstexpandierenden Stents (10) verbunden ist und sich zwischen zwei der elastischen Positionierungselemente (40) befindet; und wobei das Einströmende-Freigabeelement (60) mit dem Einströmende (1a) des selbstexpandierenden Stents (10) verbunden ist.

2. Transkatheter-Aortenklappenersatzvorrichtung mit bilateraler Verriegelungsfunktion gemäß Anspruch 1, wobei der Verbindungsarm (42) mit dem Ausströmende-Gitter (12) oder dem Ausströmende-Freigabeelement (50) an einem ersten Verbindungspunkt (L1) verbunden ist; das Ausströmende-Freigabeelement (50) mit dem Ausströmende-Gitter (12) an einem zweiten Verbindungspunkt (L2) verbunden ist; und
mindestens einer des ersten Verbindungspunkts (L1) und des zweiten Verbindungspunkts (L2) von einem Scheitelpunkt des Ausströmende-Gitters (12) versetzt ist.

3. Transkatheter-Aortenklappenersatzvorrichtung mit bilateraler Verriegelungsfunktion gemäß Anspruch 1, wobei ein eingeschlossener Winkel zwischen dem Verbindungsarm (42) und einer Ebene, die zu dem entsprechenden Ausströmende-Gitter (12) tangential ist, in einem Bereich von 0°~45° liegt;
und wobei sich optional der Verbindungsarm (42) auf der Ebene befindet, die zu dem entsprechenden Ausströmende-Gitter (12) tangential ist.

4. Transkatheter-Aortenklappenersatzvorrichtung mit bilateraler Verriegelungsfunktion gemäß Anspruch 1, wobei drei elastische Positionierungselemente (40) bereitgestellt sind und die drei elastischen Positionierungselemente (40) gleichmäßig in einer Umfangsrichtung des selbstexpandierenden Stents (10) verteilt sind.

5. Transkatheter-Aortenklappenersatzvorrichtung mit bilateraler Verriegelungsfunktion gemäß einem der Ansprüche 1 bis 4, wobei Reparaturteile (43) nahe zwei Enden des U-förmigen Abschnitts (41) gebildet sind;
die Transkatheter-Aortenklappenersatzvorrichtung ferner ein Reparaturelement beinhaltet und wobei jedes Reparaturteil (43) durch das Reparaturelement mit dem selbstexpandierenden Stent (10) verbindbar ist.

6. Transkatheter-Aortenklappenersatzvorrichtung mit bilateraler Verriegelungsfunktion gemäß Anspruch 5, wobei sich das Reparaturteil (43) und der erste Verbindungspunkt (L1) an zwei Enden des Verbindungsarms (42) befinden; das Reparaturelement eine weiche lagenartige Struktur aufweist; das Reparaturelement in der Lage ist, sich in einer Längsrichtung des Verbindungsarms (42) um den Verbindungsarm (42) zu wickeln oder zu winden; und zwei Enden des Reparaturelements, die dem Verbindungsarm (42) entsprechen, an dem entsprechenden ersten Verbindungspunkt (L1) bzw. dem entsprechenden Reparaturteil (43) befestigt sind.

7. Transkatheter-Aortenklappenersatzvorrichtung mit bilateraler Verriegelungsfunktion gemäß Anspruch 5, wobei das Reparaturelement eine längliche Streifenstruktur aufweist und ein Reparaturelement bereitgestellt ist; ein Ende des Reparaturelements in der Lage ist, sich nacheinander durch alle Reparaturteile (43) zu erstrecken und mit dem selbstexpandierenden Stent (10) verbunden zu sein; oder
wobei das Reparaturelement eine längliche Streifenstruktur aufweist; und die Anzahl der Reparaturelemente gleich der Anzahl der elastischen Positionierungselemente (40) ist; und ein Ende jedes der Reparaturelemente sich nacheinander durch die Reparaturteile (43) auf demselben U-förmigen Abschnitt (41) erstreckt und mit dem selbstexpandierenden Stent (10) verbunden ist.

8. Transkatheter-Aortenklappenersatzvorrichtung mit bilateraler Verriegelungsfunktion gemäß Anspruch 6, die ferner ein Schutzelement (44) beinhaltet, das durch Nähen, Wickeln oder Winden mit einer Außenseite eines Mittelteils des U-förmigen Abschnitts (41) verbunden ist;
und wobei optional das Reparaturelement und das Schutzelement (44) Polyestergewebe oder ein Rinderperikard sind.

9. Transkatheter-Aortenklappenersatzvorrichtung mit bilateraler Verriegelungsfunktion gemäß Anspruch 2, wobei das Ausströmende-Freigabeelement (50) einen Freigabeabschnitt (51) und Stützarme (52) beinhaltet, die sich auf zwei Seiten des Freigabeabschnitts (51) befinden; der Stützarm (52) mit dem selbstexpandierenden Stent (10) an dem zweiten Verbindungspunkt (L2) verbunden ist; sich zwei zweite Verbindungspunkte (L2) desselben Ausströmende-Freigabeelements (50) an Scheitelpunkten verschiedener Ausströmende-Gitter (12) befinden; und sich der Freigabeabschnitt (51) von dem Einströmende (1a) weg erstreckt.

10. Transkatheter-Aortenklappenersatzvorrichtung mit bilateraler Verriegelungsfunktion gemäß Anspruch 9, wobei ein Freigabegitter (53) von den Stützarmen (52) jedes Ausströmende-Freigabeelements (50) und angrenzenden Außenseiten von zwei Ausströmende-Gittern (12) umschlossen ist; ein Ende des Klappensegels (30) angrenzend an das Einströmende (1a) mit der Klappenschürze (20) in einer Umfangsrichtung vernäht ist und ein anderes Ende des Klappensegels (30) mit der Klappenschürze (20) an einer Position innerhalb des Freigabegitters (53) verbunden ist.

11. Transkatheter-Aortenklappenersatzvorrichtung mit bilateraler Verriegelungsfunktion gemäß Anspruch 10, wobei eine Lochgröße des Durchgangslochs in einer ersten Richtung (A) allmählich kleiner wird und dann unverändert bleibt oder allmählich größer wird und das Freigabegitter (53) in der ersten Richtung (A) zu einer Innenseite des Durchgangslochs hin gebogen ist.

12. Transkatheter-Aortenklappenersatzvorrichtung mit bilateraler Verriegelungsfunktion gemäß Anspruch 10, wobei das elastische Positionierungselement (40) und der selbstexpandierende Stent (10) durch Schneiden eines Nickel-Titan-Legierungsrohrs in einem einzigen Durchgang, gefolgt von Thermoformen, gebildet sind.

13. Transkatheter-Aortenklappenersatzvorrichtung mit bilateraler Verriegelungsfunktion gemäß Anspruch 1, wobei eine Seitenwand des selbstexpandierenden Stents (10) angrenzend an das Einströmende (1a) durch eine Vielzahl von Einströmende-Gittern (11) gebildet ist, die in einer Ringform angeordnet sind; und das Einströmende-Freigabeelement (60) eine elliptische Struktur (61) beinhaltet, die mit einem Scheitelpunkt eines der Einströmende-Gitter (11) verbunden ist.

14. Transkatheter-Aortenklappenersatzvorrichtung mit bilateraler Verriegelungsfunktion gemäß Anspruch 13, wobei ein Durchdringungsloch an einem Mittelteil der elliptischen Struktur (61) in einer radialen Richtung des Durchgangslochs gebildet ist.

15. Transkatheter-Aortenklappenersatzvorrichtung mit bilateraler Verriegelungsfunktion gemäß Anspruch 13, wobei eine Verlängerungslinie einer kurzen Achse der elliptischen Struktur (61) parallel zu einer Achse des Durchgangslochs ist und sich eine Verlängerungslinie einer langen Achse der elliptischen Struktur (61) auf einer Ebene befindet, die zu dem entsprechenden Einströmende-Gitter (11) tangential ist.

## Revendications

1. Un appareil de remplacement de valve aortique par transcathéter ayant une fonction de verrouillage bilatéral, comprenant une endoprothèse auto-expansible (10), une jupe de valve (20), un feuillet de valve (30), au moins deux éléments de positionnement élastiques (40), un élément de libération d'extrémité de sortie (50) et un élément de libération d'extrémité d'entrée (60) ;
dans lequel un trou traversant pour la circulation de liquide est défini à l'intérieur de l'endoprothèse auto-expansible (10) ; l'endoprothèse auto-expansible (10) a une extrémité d'entrée (1a) et une extrémité de sortie (1b) opposée à l'extrémité d'entrée (1a), correspondant au trou traversant ; et une paroi latérale de l'endoprothèse auto-expansible (10) a une structure en forme de grille ; dans lequel la jupe de valve (20) est reliée à une paroi latérale du trou traversant ; dans lequel le feuillet de valve (30) est relié à un côté interne de la jupe de valve (20) ; une paroi latérale de l'endoprothèse auto-expansible (10) adjacente à l'extrémité de sortie (1b) est formée par une pluralité de grilles d'extrémité de sortie (12) agencées selon une forme annulaire ;
chaque élément de positionnement élastique (40) a une portion en forme de U (41) et des bras de liaison (42) situés au niveau de deux extrémités de la portion en forme de U (41) ; dans lequel chaque bras de liaison (42) est relié à l'extrémité de sortie (1b) ; la portion en forme de U (41) est située sur un côté externe de l'endoprothèse auto-expansible (10) ; et une partie médiane de la portion en forme de U (41) s'étend vers l'extrémité d'entrée (1a) et est élastiquement pliable ; et
dans lequel l'élément de libération d'extrémité de sortie (50) est relié à l'extrémité de sortie (1b) de l'endoprothèse auto-expansible (10) et situé entre deux des éléments de positionnement élastiques (40) ; et l'élément de libération d'extrémité d'entrée (60) est relié à l'extrémité d'entrée (1a) de l'endoprothèse auto-expansible (10).

2. L'appareil de remplacement de valve aortique par transcathéter ayant une fonction de verrouillage bilatéral selon la revendication 1, dans lequel le bras de liaison (42) est relié à la grille d'extrémité de sortie (12) ou à l'élément de libération d'extrémité de sortie (50) au niveau d'un premier point de liaison (L1) ; l'élément de libération d'extrémité de sortie (50) est relié à la grille d'extrémité de sortie (12) au niveau d'un deuxième point de liaison (L2) ; et
au moins un point de liaison parmi le premier point de liaison (L1) et le deuxième point de liaison (L2) est décalé par rapport à un sommet de la grille d'extrémité de sortie (12).

3. L'appareil de remplacement de valve aortique par transcathéter ayant une fonction de verrouillage bilatéral selon la revendication 1, dans lequel un angle inclus entre le bras de liaison (42) et un plan tangent à la grille d'extrémité de sortie (12) correspondante est compris dans une plage de 0°~45° ;
et facultativement dans lequel le bras de liaison (42) est situé sur le plan tangent à la grille d'extrémité de sortie (12) correspondante.

4. L'appareil de remplacement de valve aortique par transcathéter ayant une fonction de verrouillage bilatéral selon la revendication 1, dans lequel trois éléments de positionnement élastiques (40) sont prévus, et les trois éléments de positionnement élastiques (40) sont uniformément répartis dans une direction circonférentielle de l'endoprothèse auto-expansible (10).

5. L'appareil de remplacement de valve aortique par transcathéter ayant une fonction de verrouillage bilatéral selon l'une quelconque des revendications 1 à 4, dans lequel des parties de réparation (43) sont formées près de deux extrémités de la portion en forme de U (41) ;
l'appareil de remplacement de valve aortique par transcathéter comprend en outre un élément de réparation, et dans lequel chaque partie de réparation (43) peut être reliée à l'endoprothèse auto-expansible (10) par le biais de l'élément de réparation.

6. L'appareil de remplacement de valve aortique par transcathéter ayant une fonction de verrouillage bilatéral selon la revendication 5, dans lequel la partie de réparation (43) et le premier point de liaison (L1) sont situés au niveau de deux extrémités du bras de liaison (42) ; l'élément de réparation a une structure de type feuille souple ; l'élément de réparation est capable d'envelopper ou de s'enrouler autour du bras de liaison (42) dans une direction de la longueur du bras de liaison (42) ; et deux extrémités de l'élément de réparation correspondant au bras de liaison (42) sont fixées au premier point de liaison correspondant (L1) et à la partie de réparation correspondante (43), respectivement.

7. L'appareil de remplacement de valve aortique par transcathéter ayant une fonction de verrouillage bilatéral selon la revendication 5, dans lequel l'élément de réparation a une structure de bande allongée et un élément de réparation est prévu ; une extrémité de l'élément de réparation est capable de s'étendre à travers toutes les parties de réparation (43) en séquence et d'être reliée à l'endoprothèse auto-expansible (10) ; ou dans lequel l'élément de réparation a une structure de bande allongée ; et le nombre des éléments de réparation est égal au nombre des éléments de positionnement élastiques (40) ; et une extrémité de chacun des éléments de réparation s'étend à travers les parties de réparation (43) sur la même portion en forme de U (41) en séquence et est reliée à l'endoprothèse auto-expansible (10).

8. L'appareil de remplacement de valve aortique par transcathéter ayant une fonction de verrouillage bilatéral selon la revendication 6, comprenant en outre un élément de protection (44) relié à un côté externe d'une partie médiane de la portion en forme de U (41) par suture, enveloppement, ou enroulement ;
et facultativement dans lequel l'élément de réparation et l'élément de protection (44) sont un tissu en polyester ou un péricarde bovin.

9. L'appareil de remplacement de valve aortique par transcathéter ayant une fonction de verrouillage bilatéral selon la revendication 2, dans lequel l'élément de libération d'extrémité de sortie (50) comprend une portion de libération (51) et des bras de support (52) situés sur deux côtés de la portion de libération (51) ; le bras de support (52) est relié à l'endoprothèse auto-expansible (10) au niveau du deuxième point de liaison (L2) ; deux deuxièmes points de liaison (L2) du même élément de libération d'extrémité de sortie (50) sont situés au niveau de sommets de différentes grilles d'extrémité de sortie (12) ; et la portion de libération (51) s'étend en s'éloignant de l'extrémité d'entrée (1a).

10. L'appareil de remplacement de valve aortique par transcathéter ayant une fonction de verrouillage bilatéral selon la revendication 9, dans lequel une grille de libération (53) est entourée par les bras de support (52) de chaque élément de libération d'extrémité de sortie (50) et des côtés externes adjacents de deux grilles d'extrémité de sortie (12) ; une extrémité du feuillet de valve (30) adjacente à l'extrémité d'entrée (1a) est suturée à la jupe de valve (20) dans une direction circonférentielle, et une autre extrémité du feuillet de valve (30) est reliée à la jupe de valve (20) au niveau d'une position à l'intérieur de la grille de libération (53).

11. L'appareil de remplacement de valve aortique par transcathéter ayant une fonction de verrouillage bilatéral selon la revendication 10, dans lequel une taille de trou du trou traversant devient progressivement plus petite et reste ensuite inchangée ou devient progressivement plus grande dans une première direction (A), et la grille de libération (53) est courbée vers un côté interne du trou traversant dans la première direction (A).

12. L'appareil de remplacement de valve aortique par transcathéter ayant une fonction de verrouillage bilatéral selon la revendication 10, dans lequel l'élément de positionnement élastique (40) et l'endoprothèse auto-expansible (10) sont formés par découpe d'un tuyau en alliage nickel-titane en un seul passage, suivie d'un thermoformage.

13. L'appareil de remplacement de valve aortique par transcathéter ayant une fonction de verrouillage bilatéral selon la revendication 1, dans lequel une paroi latérale de l'endoprothèse auto-expansible (10) adjacente à l'extrémité d'entrée (1a) est formée par une pluralité de grilles d'extrémité d'entrée (11) agencées selon une forme annulaire ; et l'élément de libération d'extrémité d'entrée (60) comprend une structure elliptique (61) reliée à un sommet d'une des grilles d'extrémité d'entrée (11).

14. L'appareil de remplacement de valve aortique par transcathéter ayant une fonction de verrouillage bilatéral selon la revendication 13, dans lequel un trou de pénétration est formé au niveau d'une partie médiane de la structure elliptique (61) dans une direction radiale du trou traversant.

15. L'appareil de remplacement de valve aortique par transcathéter ayant une fonction de verrouillage bilatéral selon la revendication 13, dans lequel une ligne d'extension d'un axe court de la structure elliptique (61) est parallèle à un axe du trou traversant, et une ligne d'extension d'un axe long de la structure elliptique (61) est située sur un plan tangent à la grille d'extrémité d'entrée (11) correspondante.
